# EUROPEAN PATENT APPLICATION

(11) **EP 4 840 935 A1**
(43) Date of publication of application: **18.11.2026**
(21) Application number: 25182068.4
(22) Date of filing: 11.06.2025
(51) Int. Cl.: C12M 1/00, C12M 1/26, C12M 1/34, C12M 1/32, C12M 1/36

(54) **PHAGE ENRICHMENT AND CLONAL ISOLATION SYSTEM AND METHODS THEREOF**

(30) Priority: 15.05.2025 EP 25176677; 15.05.2025 EP 25176688
(71) Applicant: Medea Biopharma GmbH, 67059 Ludwigshafen am Rhein (DE)
(72) Inventor: Trojok, Rüdiger, 76879 Essingen (DE)
(74) Representative: Stellbrink & Partner Patentanwälte mbB

(57) **Abstract**

The present invention relates to a system and method for the continuous and automated production and isolation of bacteriophages. It is preferably related to leveraging an integrated bioreactor comprising multiple compartments for bacterial culture, bacteriophage enrichment and clonal isolation. The invention also describes multiple applications of the produced phages and derivatives thereof in medical, veterinary, agricultural, and industrial fields.

## Description

The invention relates to the fields of microbiology and biotechnology, specifically to a system and methods for the production, isolation, and application of bacteriophages (phages). The invention further includes applications of these phages in various industries including healthcare, veterinary medicine, agriculture, farming, environmental sanitation and other.

Phages are viruses that specifically infect and replicate within bacteria. They are composed of proteins that encapsulate a DNA or RNA genome. Phages are highly diverse and can be found in any environment where bacteria exist, making them the most abundant biological entities on Earth. They play a significant role in regulating bacterial populations in natural environments and have various applications, including their use in phage therapy to treat bacterial infections that are resistant to antibiotics.

However, the scalability of phage production and the specificity required for various applications pose significant challenges. Existing methods often rely on labour-intensive batch processes, which limit the ability to produce large quantities of high-purity phages. There have been developments in this field that aimed to address these limitations. For instance, WO 2014/001504 A2 describes that bacteriophages are produced in a continuous process comprising maintaining a culture of bacteriophage in a first vessel; feeding the culture with bacteria maintained in a second vessel in the stationary phase and harvesting bacteriophage from the first vessel, wherein the first vessel is operated at a dilution rate in excess of 1 / (bacteriophage lysis time). However, significant limitations remain. WO 2014/001504 A2 seems to be geared towards reproducing known phages, rather than isolating novel phages from a natural source.

The invention seeks to preferably address this limitation by providing a phage production system that may allow for the cultivation and isolation of phages in a way that can specifically target and effectively lyse selected bacterial strains.

The present invention, as set forth in claim 1, provides a phage system that can provide an isolation compartment where particularly effective phages can be identified and isolated using techniques such as serial dilution, liquid handling systems, and/or microfluidic devices. In serial dilution, for example, the phage solution can be diluted until finally a single phage clone that shows effective lysis remains per unit volume and is isolated. The isolated effective phages can then be cultured to establish a clonal population, where all phages are descendants of the initially effective phage.

Such a phage system comprising isolation module ensures that only the desired phage strains that are effective against specific bacteria are cultivated and proliferated. This is crucial for therapeutic and diagnostic applications where specificity and efficacy are necessary.

Further preferred and advantageous embodiments of the invention comprise the provision of at least one compartment configured for phage production, enabling clonal expansion of phages under controlled conditions, with inlets for introducing growth media, bacterial cell sources or clonal phages from the isolation module, and outlets for transferring the expanded phages, thereby supporting continuous and scalable phage propagation.

A further compartment may be configured for phage harvesting, with designated inlets and outlets for streamlined downstream processing of expanded phages, enhancing operational efficiency.

The system may additionally comprise an integrated monitoring system that enables measurement of bacterial growth or lysis and the system comprises pumps configured for pumping liquid media through the system or parts thereof, and valves configured to be opened or closed, wherein the pumps and valves are controlled by signals from a control unit, and wherein the control unit is further configured to monitor the operational status of the pumps and valves, as well as at least one of temperature, pH, and nutrient supply, the system operating within an enclosed environment.

Integration with a control unit allows for automated process regulation, including the activation of pumps and valves via corresponding control signals to manage liquid flow and routing within the system.

The isolation module may be configured to perform a limited dilution series of bacteriophages, down to a dilution of 10⁻¹⁴, enabling high-resolution separation of individual phage clones.

The compartment configured for phage harvesting may comprise purification technologies such as tangential flow filtration and/or size-exclusion chromatography, facilitating efficient concentration and refinement of phage populations.

The invention also encompasses a method for continuous production of clonal phages, involving sequential steps of culturing bacterial cells, enriching phages, isolating clonal populations using microfluidic or robotic systems, and selecting the most diluted sample showing bacterial lysis for expansion.

This method enables reproducible and high-throughput clonal isolation, particularly when phages are distributed into multi-well plates (e.g., 24 to 1536 wells), allowing real-time monitoring of bacterial concentration and lysis.

The resulting clonal phage isolates, including their virions and derivatives, may be used to prepare a phage biobank which is a valuable resource of characterized isolates for future therapeutic or biotechnological applications.

Moreover, the clonal isolates, their derivatives, and associated substances as well as genetic information derived from the isolates may be employed in various therapeutic contexts, including prophylactic, metaphylactic, personalized, non-personalized, and fit-for-purpose treatments of bacterial infections, thereby contributing to the development of precise and robust antimicrobial solutions.

A phage system equipped with a bioreactor designed to manage and cultivate phages provides a controlled environment for phage growth and manipulation, enabling efficient production and study of phages under optimal conditions.

This bioreactor includes multiple compartments, allowing for the simultaneous processing of different phage cultures or stages of development, enhancing the system's versatility and throughput.

One of these compartments is specifically configured for culturing bacterial cells, facilitating direct cultivation of target bacteria and immediate interaction essential for phage replication.

The compartment designed for culturing bacterial cells includes at least one inlet to facilitate the controlled introduction of growth media and bacterial cells, maintaining the culture without compromising the sterile environment or the experimental setup integrity.

The inlets are configured to introduce growth media and bacterial cell sources efficiently, promoting optimal growth conditions and effective phage infection cycles.

A monitoring system within the compartment for culturing bacterial cells enables real-time observation and measurement of parameters critical to successful bacterial cultivation and phage production.

The monitoring system is configured to measure bacterial cell growth, optimizing phage production protocols by accurately tracking the growth rates of bacterial cultures.

Various physical or chemical detection methods are used to measure bacterial cell growth, including OD measurement, LED measurement, light interaction fluorescence, Nanoparticle Tracking Analysis, Attenuated Total Reflection Infrared spectroscopy, flow cytometry, calorimetric measurement, photo diode measurement, and visual microscope image analysis. These methods provide comprehensive data to fine-tune the cultivation environment.

In particular, the monitoring system is set up for measuring optical density (OD), a method for assessing bacterial cell concentration and growth dynamics.

A spectrophotometer employed in the OD monitoring system provides accurate and reliable measurements, crucial for maintaining control over growth conditions within the bioreactor.

The bioreactor also includes at least one outlet, complementing the inlet systems and allowing for the efficient removal or transfer of cultured bacterial cells, facilitating cell density management and further processing or analysis.

The outlets are specifically configured for transferring cultured bacterial cells, integrating the bacterial cultures into subsequent phage production stages or for harvesting.

With two compartments configured for culturing bacterial cells, the bioreactor allows for parallel culturing of different bacterial strains or conditions, increasing the system's flexibility and experimental capacity.

Expanding further, the bioreactor includes three compartments for culturing bacterial cells, increasing throughput and at its most expanded, the bioreactor comprises four or more compartments for culturing bacterial cells, maximizing experimental capacity and enabling extensive studies with multiple bacterial strains or varied environmental conditions.

Each compartment is capable of housing different bacterial strains or species, essential for phage research and production as it facilitates the exploration of phage-host interactions across diverse bacterial backgrounds.

The bioreactor's design incorporates at least one compartment specifically for phage enrichment, providing a dedicated environment that supports selective cultivation and amplification of phages.

This compartment includes at least one inlet, similar to those in the bacterial culturing compartments, which allows for the controlled introduction of materials necessary for initiating and maintaining phage enrichment processes.

The inlets of the phage enrichment compartment are configured to introduce an initial population of phages, along with growth media and bacterial cells for replenishment, ensuring continual support for phage cultivation and amplification.

Capable of housing a mixture of different phages, the compartment allows for the cultivation of multiple phage types simultaneously, facilitating studies on phage interactions and the development of phage cocktails.

Phages can be sourced from well-characterized, clonal laboratory stock cultures, enabling controlled studies with defined phage types. This facilitates reproducible experiments and the reliable scale-up of phage production for therapeutic or biocontrol applications. In contrast, if a mixture of phages produced in the lab is used, prior isolation and characterization steps are necessary before such controlled production can be achieved.

Alternatively, the mixture in the compartment configured for phage enrichment can be derived from environmental and clinical samples, valuable for discovering new phages with potentially unique properties or broader host ranges.

Specifically, environmental samples such as filtered wastewater provide a rich microbial diversity, offering a robust resource for novel phage isolation.

Another source for the compartment configured for phage enrichment are swabs e.g. taken from excretions or in the form of a watery extract, providing diverse microbial communities and increasing the likelihood of discovering phages with unique capabilities or broad-spectrum activity.

In some cases, the mixture of phages for the compartment configured for phage enrichment can come from premixed or premanufactured phage preparations, allowing for controlled study of specific phage combinations and the development of tailored phage therapies.

Equipped to house bacterial cells, the compartment configured for phage enrichment ensures that phages have access to necessary hosts within the same compartment, facilitating efficient infection cycles and phage propagation.

Bacterial cells within the compartment configured for phage enrichment can be sourced from compartments configured for culturing bacterial cells, maintaining consistency and continuity of bacterial cells used throughout the cultivation stages.

Further developed, the compartment configured for phage enrichment incorporates monitoring systems capable of tracking and analyzing phage and bacterial cell interactions, providing valuable data on phage activity and enrichment process effectiveness.

Configured to measure the reduced growth rate of bacteria due to phage lysis, the monitoring system within the compartment configured for phage enrichment is essential for determining phage replication timing and bacterial cell destruction extent.

Based on obtained measurements, phage growth can be inferred from the observed decrease in live bacterial concentration, providing a direct method for assessing enrichment process success and phage potency.

Including at least one outlet, similar to those in the bacterial culturing compartments, the compartment configured for phage enrichment facilitates material removal or transfer, useful for harvesting enriched phages or removing waste products.

With two compartments configured for phage enrichment, the bioreactor allows for parallel enrichment of different phage populations or separation of processes under varying conditions, enhancing throughput and flexibility.

Further expanded, the bioreactor includes three compartments configured for phage enrichment, supporting more complex experiments or simultaneous multiple sample processing, significantly increasing capacity for phage production and research.

At its most extensive, the bioreactor comprises four or more compartments configured for phage enrichment, maximizing handling capacity for diverse phage types or conducting large-scale studies, offering substantial flexibility and scalability in phage production.

Including at least one isolation module, the phage system provides a critical component for the precise isolation and handling of individual phages or phage populations, essential for maintaining phage culture purity and specificity, particularly in applications requiring high precision and control.

The isolation module includes equipment such as a pipetting robot, a digital microfluidic system, or a gradient microfluidic chip, providing advanced capabilities for handling and manipulating small fluid volumes, essential for precise phage and bacterial culturing.

Configured to perform a limited dilution series of bacteriophages until a dilution of 10^-14 is achieved, the isolation module allows for isolating single phage particles or clonal phage populations, ensuring genetically uniform phages for further experiments or applications.

Including at least one inlet configured for receiving a filtered mixture of phages from the compartment configured for phage enrichment, the isolation module ensures that only desired phages, free from unwanted particles or contaminants, are introduced, maintaining the purity and specificity of the isolation process.

Additionally, the isolation module includes an inlet for receiving bacterial cells from the compartment configured for culturing bacterial cells, facilitating immediate and effective phage infection experiments within the module.

Also including an inlet for receiving buffer or media from an external source, the isolation module ensures that necessary reagents and media are introduced, conducive to phage activity and bacterial growth.

Including one or more dead-end filters, at least one of the inlets of the isolation module prevents contamination and ensures that only intended substances are introduced, maintaining experiments' sterility and integrity.

The filters within the isolation module, with a pore size range from 0.10 µm to 1.0 µm, effectively filter small particles and contaminants without obstructing the flow of smaller molecules and ions essential for phage and bacterial activity.

Preferably, the filters have a pore size of 0.22 µm to 0.45 µm, providing an optimal balance between filtration efficiency and flow rate, effective without significantly slowing down fluid processing within the module.

More preferably, the filters have a pore size of 0.22 µm, particularly effective for the applications envisioned with this phage system, providing an ideal level of filtration to maintain phage culture purity and quality.

Connected in series, the filters within the isolation module enhance filtration efficiency by sequentially reducing contaminants on each subsequent filter, extending filter life and improving overall filtration quality.

Comprising a pipetting robot, the isolation module automates handling and precise delivery of small liquid volumes, crucial for setting up and executing experiments within the module.

Further comprising or alternatively comprising a digital microfluidic device that utilizes electrowetting on dielectric to create, merge, and manipulate droplets, this device allows precise control of fluidic operations at the microscale, essential for detailed study and manipulation of phage and bacterial interactions.

Including several droplets generated on the digital microfluidic device, this device facilitates a serial dilution by fission of phage-containing droplets and merging them with droplets containing cells, buffer, or media, allowing precise control of dilution ratios and effective isolation of phage populations within the module.

Comprising a gradient microfluidic device designed to gradually dilute phage liquid using continuous liquid flow, the microfluidic channel layout in the isolation module guides the flows, allowing precise control over the dilution process and effective management of fluid dynamics.

Further comprising one or several inlets with controlled continuous liquid flow, this device configures these inlets to merge phage-containing and non-phage-containing liquids using diffusion and turbulent flows, allowing precise mixing and dilution process management, essential for controlled phage cultivation and isolation.

Comprising one or more of a pipetting robot, a digital microfluidic device, and a gradient microfluidic device, in any combination, the isolation module offers flexible and effective manipulation of fluids and cultures, catering to a wide range of experimental needs and setups.

Further comprising standard microplate formats such as 96-well or 384-well plates, the isolation module uses these formats in biological and chemical experiments, providing a convenient and standardized way to handle multiple samples simultaneously, enhancing throughput and efficiency.

Including means for continuous bacteria measurement, as previously defined in the monitoring systems of other embodiments, the isolation module tracks bacteria and phage interactions, providing real-time data on bacterial viability and phage activity.

Providing a plate reader configured to shake and incubate the well plates while measuring using a spectrophotometric plate reader which continuously monitors bacterial and phage cultures under dynamic conditions, optimizing experimental conditions and outcomes.

Configured to identify the most diluted sample that still results in bacterial lysis, the isolation module determines the minimum effective concentration of phages, providing critical information for phage therapy development and phage-bacterial interaction study.

Also configured to handle phage isolation for multiple bacterial hosts simultaneously, the isolation module processes different bacterial strains or conditions simultaneously, significantly enhancing flexibility and experimental capacity.

Further comprising an outlet provided with a filter unit, this device controls the transfer of filtered, clonally isolated phages out of the module, ensuring that only desired phage populations are collected and used in subsequent processes or studies.

Comprising a dead-end filter within the outlet filter unit, as defined in previous embodiments, the isolation module ensures that only clonal isolate phages are allowed to pass, maintaining phage population purity being transferred.

Including at least one compartment configured for phage production, the bioreactor provides a dedicated environment for the clonal expansion of phages, controlling growth and multiplication of phage populations under optimal conditions.

Specifically configured to support the clonal expansion of phages, the compartment configured for phage production ensures that phages are cultivated to promote rapid and efficient multiplication, providing a reliable phage source for research and application.

Including at least one inlet, the phage production compartment facilitates the introduction of growth media, bacterial cell sources, and isolated clonal populations of phages from the isolation module, ensuring necessary components for phage production are readily available.

Configured to introduce growth media, bacterial cell sources, and filtered isolated clonal populations of phages, the inlets of the compartment configured for phage production ensure successful phage cultivation by introducing all necessary inputs in a controlled and efficient manner.

Also including a monitoring system, as defined in earlier embodiments, the compartment configured for phage production tracks and analyzes various parameters, providing valuable data for optimizing phage production and ensuring bacterial viability.

Configured to measure a reduced growth rate of bacteria due to phage lysis, the monitoring system in the compartment configured for phage production is essential for determining the effectiveness of phages in infecting and lysing bacterial cells.

Based on data from the monitoring system, phage growth within the compartment configured for phage production, is inferred from the observed decrease in live bacterial concentration, providing a straightforward method for assessing phage cultivation progress and production process success.

Including at least one outlet, the compartment configured for phage production facilitates efficient phage removal or transfer, supporting the harvesting of phages for further processing or application.

Configured for recirculating phages to the compartment configured for phage enrichment, the outlet of the compartment configured for phage production minimizes waste and maximizes phage cultivation productivity.

Providing a filter in the outlet between the compartment configured for phage production and the compartment configured for phage enrichment that removes bacterial cells, ensures that only phages are recirculated.

Including a compartment configured for phage harvesting, the bioreactor collects clonal phages from the phage production compartment, providing an effective separation and purification environment for phages from other components.

Including a tangential flow filtration system, the compartment configured for phage harvesting separates clonal phages from bacterial cells and culture medium, using a membrane-based process to retain larger particles while allowing phages to pass.

Alternatively including a filtration system for concentrating and purifying clonal phages, the compartment configured for phage harvesting uses filters or membranes to separate phages from larger or smaller particles, such as bacterial cells and debris, ensuring only desired phage particles are collected.

Using a microfiltration system, the filtration system in the compartment configured for phage harvesting separates clonal phages from larger or smaller particles, providing a high degree of purity in the harvested phage populations.

Also including a size-exclusion chromatography system, the compartment configured for phage harvesting purifies and separates clonal phages from contaminants based on their size, using a gel or other matrix-filled column to selectively allow smaller or larger particles to pass at different rates.

Further including at least one clonal phage collecting container, the phage system collects purified phages after passing through harvesting processes, providing a controlled environment for phage population storage and handling.

Including an outlet specifically configured for feeding back the retentate to the compartment configured for phage production, the compartment configured for phage harvesting enhances the efficiency and yield of the phage cultivation system by recirculating any unharvested phages or other valuable components back into the production process.

Specifically configured pumps within the phage system for pumping liquid media through the system or through specific parts thereof, are essential for moving and managing fluids within the system, facilitating the transfer and mixing of growth media, bacteria, and other components.

Including both syringe pumps and peristaltic pumps, each selected based on the volume of liquids to be pumped, the phage system uses syringe pumps for smaller, more precise volumes, while peristaltic pumps are used for larger volumes, providing flexibility and precision in handling various liquid volumes within the system.

Configured to pump dosage amounts in the range of 10 to 2500 µl, the syringe pumps within the phage system are used for dosing bacteria growth media, bacterial cell sources, phage samples, and other reagents, providing precise control over the amounts of each component introduced into the system.

Configured to pump volumes greater than 2500 µl, the peristaltic pumps within the phage system are typically used for handling larger volumes of liquid, such as water, cleaning solutions, waste, and phage products, ensuring efficient and effective management of these substances within the system.

Handling larger volumes of liquid, including water, cleaning solutions (such as NaOH), waste, and phage products, the larger volumes managed by the peristaltic pumps are essential for the maintenance, cleaning, and operation of the phage system, and their effective management is critical for maintaining the system's performance and cleanliness. Configured for controlling the flow of liquid media and the volume thereof within one module and through the different modules, units, and compartments, the valves within the phage system play a crucial role in directing the movement of fluids within the system, ensuring that each liquid is delivered to the right place at the right time.

Managing liquid media, including bacteria growth media, bacterial cell sources, and phage samples from the various compartments and modules of the phage system, the valves controlled the flow of these media, optimizing the environmental conditions within each part of the system for the tasks being performed.

Configured to be opened or closed upon receiving a corresponding signal from the control unit, the valves within the phage system allow for precise and timely adjustments to the flow of liquids within the system, ensuring smooth and effective operation according to the programmed protocols.

A method involves the continuous and automated isolation of clonal phage populations from a phage source containing a mixture or a single species of phages. This enhances the efficiency and reproducibility of obtaining specific phage clones for detailed study or applications.

The method uses a liquid phage source that may contain a mixture of natural, artificial, or combinations of different types of phages. Using a liquid medium facilitates the manipulation and processing of phages during isolation.

Where the liquid phage source includes a mixture of known or unknown phages, it allows for broad exploration of phage diversity, enhancing the potential to discover phages with unique properties or broader applications.

Often, an environmental, laboratory stock, or clinical sample serves as a phage source. This diversity in source material broadens the scope of phage types available for research and application, increasing the method's versatility.

A wastewater sample is used as an environmental phage source in some approaches. Filtering this wastewater before use ensures the removal of unwanted particles, which might interfere with isolation processes.

The method incorporates the use of one or more dead-end filters with specific pore sizes to ensure the cleanliness of the phage samples being processed. These filters effectively remove larger contaminants while allowing phages to pass through.

Phage enrichment processes are applied, where the filtered phage source is added to containers containing bacterial cultures. This setup enables phages to interact directly with potential host bacteria, facilitating natural selection and propagation of effective phage variants.

When multiple compartments configured for phage enrichment are used, each containing different bacterial cultures, this allows for simultaneous testing and optimization of phage-host interactions under varied conditions, significantly enhancing the breadth of data collected.

The growth of bacterial cultures within these containers is carefully monitored, maintaining the cultures at optimal conditions for phage infection, which is critical for effective phage amplification and isolation.

The method ensures that the bacteria are grown to either an exponential or a stationary phase, depending on the desired phage interaction dynamics, providing control over the infection cycle and phage production rates.

Bacteria used in these processes are often isolated from diagnostic laboratories, providing a controlled source of epidemiologically relevant bacteria that enhance the applicability of the phages in medical or industrial settings.

The system keeps the bacterial growth in a steady state by adding fresh media while transferring outgrown cultures to maintain the culture's viability, which is crucial for sustained phage production.

Bacteria are transferred either continuously or in batches to new containers or modules at concentrations optimized for phage infection, ensuring efficient use of resources and maximizing phage yield.

Physical or chemical detection methods such as OD measurement, LED measurement, or flow cytometry are used to quantify bacteria concentrations. This provides accurate data for fine-tuning the phage enrichment process and assessing the effectiveness of different phage strains.

When a reduced growth rate of bacteria due to phage lysis is observed, this indicates successful phage enrichment. Monitoring these changes allows for the timely extraction and further processing of effective phages.

The enriched phage mixture undergoes a clonal isolation process, potentially involving a technique that uses a limited dilution series to isolate single phage clones. This method is crucial for obtaining genetically pure phage populations for detailed study or therapeutic application.

The isolation of phage populations involves distributing the enriched phage mixture into wells of a microplate. This setup allows for precise control over the volume and concentration of phages in each well, facilitating the isolation of individual phages based on their ability to infect and lyse bacteria.

Microplates used in these methods may vary in size, from 24 to 1536 wells, allowing for scalability and flexibility in the number of phage isolations performed simultaneously. A digital microfluidics technique using electrowetting on dielectric principles is sometimes employed to isolate phages. This method manipulates small droplets on a surface to separate phage populations based on their physical properties and interactions with bacterial cells.

Alternatively, a continuous flow microfluidics chip is used for phage isolation. This chip allows for the mixing of phages and bacteria in a controlled flow, facilitating the observation and selection of phages that effectively lyse bacteria at the lowest dilution.

Once isolated, phage populations are transferred to a compartment configured for phage production where they are grown in the presence of host bacterial cells. This step is crucial for amplifying the phages to quantities sufficient for further study or application.

The production of phages is closely monitored, with conditions such as optical density (OD) being measured to determine the optimal time for harvesting the phages based on their concentration and activity.

Phages are harvested using techniques such as tangential flow filtration or microfiltration, which separate phages from bacterial cells and other particles, ensuring the collection of high-purity phage samples for downstream processes.

The harvested phages may undergo further processes such as up-concentration or recycling to enhance their concentration and purity before being used in further applications or studies.

The entire phage production system may be subjected to cleaning processes involving strong solutions like NaOH followed by thorough rinsing with water, ensuring the removal of residues and the maintenance of a sterile environment for subsequent phage production cycles.

This method not only facilitates the effective production and isolation of phages but also ensures their applicability across various fields, from medical therapeutics to environmental management, by providing robust, scalable, and flexible processes tailored to specific phage properties and application needs.

The use of at least one clonal phage isolate, including its virions and and respective (genetic) information, in the preparation of a phage biobank, represents a structured approach to storing characterized phage isolates for future applications. This phage biobank serves as a repository, ensuring that a diverse range of phages is available for research and practical use.

The phage biobank not only stores phage isolates but also includes phage-derived substances and comprehensive information about the phages. This information and material enrich the biobank's utility for scientific and therapeutic endeavors.

Among the phage-derived substances included in the biobank are proteins, such as enzymes like endolysins and holins. These enzymes are critical in the study of phage lytic cycles and have applications in developing antibacterial agents.

The phage-derived information stored includes genomic data, such as nucleic acid sequences, which are essential for genetic studies, phage engineering, and tracking phage evolution.

Additionally, the biobank captures phage phenotypical characteristics, which are invaluable for understanding phage behaviour, infection patterns, and suitability for specific applications.

The resources from the biobank are used in the preparation of pharmaceutical active ingredients for both human and veterinary medicine, showcasing the medical potential of phages in treating bacterial infections.

In a specific application, these resources are utilized in the development of medical devices designed to deliver phage-based treatments directly to infection sites, offering targeted therapeutic options.

The phage biobank also supports extensive research and product development activities, where phages are analyzed to innovate new therapeutic and diagnostic tools, enhancing our understanding of microbial interactions and phage biology.

Phages and their derivatives are applied in various industrial applications, reflecting their versatility. These applications include the food industry, agriculture, and other sectors such as cosmetics and water management, where phages play roles in biocontrol and maintaining hygiene.

Specifically, in industries like food processing, food supplements, farming and agriculture, phages are used as biocides and preservatives, providing non-chemical options for pathogen control.

The biobank's resources are employed in diagnostics outside the human and animal body, offering methods for detecting and monitoring bacterial populations in environmental and clinical settings.

In biotechnology, the phage resources facilitate the development of new biotechnological tools and processes, potentially transforming genetic engineering and synthetic biology.

Phage isolates are used in various therapies such as prophylactic, metaphylactic, personalized, and non-personalized therapies, aimed at preventing or treating infectious diseases in humans and animals, demonstrating the therapeutic versatility of phages.

These therapies are particularly pertinent for individuals, whether humans or animals, dealing with infections caused by antibiotic-resistant bacteria or commonly encountered pathogens.

The targeted bacteria in these therapies include antibiotic-resistant strains such as carbapenem-resistant Klebsiella pneumoniae, Colistin-, Carbapenem- and panresistant *Pseudomonas aeruginosa* and methicillin-resistant Staphylococcus aureus, reflecting the critical role of phages in addressing antibiotic resistance.

The scope of application extends to treating infections in companion animals and food-producing animals, caused by bacteria such as Staphylococcus spp., *Pseudomonas aeruginosa, Proteus spp.,* Escherichia coli, and Salmonella spp., emphasizing the broad utility of phage therapy in veterinary medicine.

The invention is also defined by the following numbered embodiments:
Below, product, method and use embodiments will be discussed. These embodiments are abbreviated by letters "P", "M" and "U" followed by a number.

| | |
|---|---|
| P1. | A phage system comprising a bioreactor. |
| P2. | The phage system according to P1 wherein the bioreactor comprises a plurality of compartments. |
| P3. | The phage system according to P2 wherein the plurality of compartments comprises at least one compartment configured for culturing bacterial cells. |
| P4. | The phage system according to P3 wherein the at least one compartment for culturing bacterial cells comprises at least one inlet. |
| P5. | The phage system according to P4 wherein the inlet/s is/are configured for introducing growth media and/or bacterial cell source. |
| P6. | The phage system according to at least one of P3 to P5, wherein the at least one compartment configured for culturing bacterial cells further comprises a monitoring system. |
| P7. | The phage system according to P6, wherein the monitoring system is configured for measuring bacterial cell growth. |
| P8. | The method according to P7 wherein measurement of the bacterial cell growth is based on physical or chemical detection methods selected from OD measurement, LED measurement, light interaction fluorescence, Nanoparticle Tracking Analysis, Attenuated Total Reflection Infrared spectroscopy, flow cytometry, calorimetric measurement, photo diode measurement and/or visual microscope image analysis. |
| P9. | The phage system according to P8, wherein the monitoring system is configured for measuring optical density (OD). |
| P10. | The phage system according to P9, wherein the OD monitoring system is a spectrophotometer. |
| P11. | The phage system according to at least one of P1 to P10, wherein the at least one compartment further comprises at least one outlet. |
| P12. | The phage system according to P11, wherein the at least one outlet is configured for transferring the cultured bacterial cells. |
| P13. | The phage system according to at least one of P3 to P12, wherein the bioreactor comprises two compartments configured for culturing bacterial cells. |
| P14. | The phage system according to P13, wherein the bioreactor comprises three compartments configured for culturing bacterial cells. |
| P15. | The phage system according to P14, wherein the bioreactor comprises four or more compartments configured for culturing bacterial cells. |
| P16. | The phage system according to at least one of P13 to P15, wherein the compartments for culturing bacterial cells comprise different bacterial strains or species. |
| P17. | The phage system according to at least one of P1 to P16, wherein the plurality of compartments comprises at least one compartment configured for phage enrichment. |
| P18. | The phage system according to P17, wherein the compartment for phage enrichment comprises at least one inlet. |
| P19. | The phage system according to P18, wherein the inlet/s is/are configured for introducing an initial population of phages, growth media and replenishing bacterial cells. |
| P20. | The phage system according to at least one P17 to P19, wherein the at least one phage enrichment compartment comprises a mixture of phages. |
| P21. | The phage system according to P20, wherein the mixture of phages comprised in the at least one compartment for phage enrichment is from a laboratory stock culture. |
| P22. | The phage system according to P20, wherein the mixture of phages comprised in the at least one compartment for phage enrichment is from an environmental or clinical sample. |
| P23. | The phage system according to P22, wherein the environmental sample is filtered wastewater. |
| P24. | The phage system according to P22, wherein the environmental sample is soil in the form of a watery extract from soil and the clinical sample is liquid or solid. |
| P25. | The phage system according to P20, wherein the mixture of phages is from a premixed or premanufactured phage preparation. |
| P26. | The phage system according to at least one of P17 to P25, wherein the compartment for phage enrichment further comprises bacterial cells. |
| P27. | The phage system according to P26, wherein the bacterial cells are from the at least one compartment configured for culturing bacterial cells. |
| P28. | The phage system according to P27, wherein the at least one compartment for phage enrichment further comprises the monitoring system of P6 to P10. |
| P29. | The phage system according to P28, wherein the monitoring system is configured to measure a reduced growth rate of bacteria due to phage lysis. |
| P30. | The phage system according to P29, wherein phage growth is inferred from a decrease in live bacterial concentration. |
| P31. | The phage system according to at least one of P17 to P30, wherein the at least one compartment for phage enrichment further comprises an outlet. |
| P32. | The phage system according to at least one of P17 to P31 wherein the bioreactor comprises two compartments for phage enrichment. |
| P33. | The phage system according to P32, wherein the bioreactor comprises three compartments for phage enrichment. |
| P34. | The phage system according to P33, wherein the bioreactor comprises four or more compartments for phage enrichment. |
| P35. | The phage system according to at least one of P1 to P34, further comprising at least one isolation module. |
| P36. | The phage system according to P35, wherein the at least one isolation module comprises a pipetting robot, a digital microfluidic system or a gradient microfluidic chip. |
| P37. | The phage system according to P36, wherein the at least one isolation module is configured to perform a limited dilution series of bacteriophages until a dilution of 10^-14. |
| P38. | The phage system according to any one of P35 to P37, wherein the at least one isolation module comprises an inlet configured for receiving a filtered mixture of phages from the compartment for phage enrichment. |
| P39. | The phage system according to P38, wherein the at least one isolation module comprises an inlet for receiving bacterial cells from the compartment for culturing bacterial cells. |
| P40. | The phage system according to P39, wherein the at least one isolation module comprises an inlet for receiving buffer or media from an external source. |
| P41. | The phage system according to at least one of P38 to P40, wherein at least one of the inlets comprises one or more dead end filters. |
| P42. | The phage system according to P41, wherein the filter/s have pore size of 0.10 µm to 1.0 µm. |
| P43. | The phage system according to P42, wherein the filter/s preferably have pore size of 0.22 µm to 0.45 µm. |
| P44. | The phage system according to P43, wherein filter/s more preferably have pore size of 0.22 µm. |
| P45. | The phage system according to P41 to P44 wherein the filters are connected in series. |
| P46. | The phage system according to P45, wherein the at least one isolation module comprises a pipetting robot. |
| P47. | The phage system according P35 wherein the at least one isolation module comprises a digital microfluidic device using electrowetting on dielectric to create, merge and manipulate droplets. |
| P48. | The phage system according to P47 wherein the isolation module further comprises one or several droplets generated on the digital microfluidic device which allow for a serial dilution by fission of phage containing droplets and merging them with cell, buffer or media containing droplets. |
| P49. | The phage system according to any one of P35 to P45 wherein the at least one isolation module comprises a gradient microfluidic device conjured to gradually dilute phage liquid using continuous liquid flow and a microfluidic channel layout to guide the flows. |
| P50. | The phage system according to at least one of P49 wherein the isolation module further comprises one or several inlets with a controlled continuous liquid flow configured to merge phage containing and non-phage containing liquids by using diffusion and turbulent flows. |
| P51. | The phage system according to any one of embodiments P46 to P50, wherein the at least one isolation module comprises one or more of a pipetting robot, a digital microfluidic device, and a gradient microfluidic device, in any combination. |
| P52. | The phage system according to P46, wherein the isolation module further comprises standard microplate formats such as 96-well or 384-well plates. |
| P53. | The phage system according to P46, wherein the isolation module comprises means for continuous bacteria measurement as defined in at least one of the P6 to P10 to detect lysis. |
| P54. | The phage system according to P46, wherein a plate reader is provided configured to shake and incubate the well plates while measuring using a spectrophotometer of P10. |
| P55. | The phage system according to P46 to P50, wherein the at least one isolation module is configured to identify the most diluted sample that still results in bacterial lysis. |
| P56. | The phage system according to P46 to P50, wherein the at least one isolation module is configured to handle phage isolation for multiple bacterial hosts simultaneously. |
| P57. | The phage system according to P55 wherein the isolation module further comprises an outlet provided with a filter unit. |
| P58. | The phage system of P57 wherein the filter unit comprises a dead-end filter as defined in P41 to P45 and/or wherein the outlet is configured for transferring filtered clonally isolated phages. |
| P59. | The phage system according to at least one P1 to P58, wherein the plurality of compartments further comprises at least one compartment for phage production. |
| P60. | The phage system according to P59, wherein the compartment for phage production is configured for clonal expansion of phages. |
| P61. | The phage system according to P59 and/or P60, wherein the compartment for phage production comprises at least one inlet. |
| P62. | The phage system according to P61, wherein the inlet/s is/are configured for introducing growth media, bacterial cell source and filtered isolated clonal population of phages from the isolation module. |
| P63. | The phage system according to at least one P59 to P62, wherein the compartment further comprises a monitoring system as defined in least one of P6 to P10. |
| P64. | The phage system according to P63, wherein the system is configured to measure a reduced growth rate of bacteria due to phage lysis. |
| P65. | The phage system according to P64, wherein phage growth is inferred from a decrease in live bacterial concentration. |
| P66. | The phage system according to at least one of P59 to P65, wherein the compartment for phage production further comprises an outlet. |
| P67. | The phage system according to P66 wherein the compartment of phage production further comprises an outlet configured for recirculating the phages to the phage enrichment compartment. |
| P68. | The phage system according to P67 wherein a filter is provided in the outlet between the phage production compartment and the phage enrichment compartment. |
| P69. | The phage system according to at least one P1 to P68, wherein the bioreactor further comprises a compartment configured for phage harvesting for collecting clonal phages from the phage production compartment. |
| P70. | The phage system according to P69, wherein the compartment configured for phage harvesting comprises a tangential flow filtration system configured for separating clonal phages from bacterial cells and culture medium. |
| P71. | The phage system according to P69, wherein the compartment configured for phage harvesting comprises a filtration system for concentrating and purifying the clonal phages. |
| P72. | The phage system according to P71, wherein the filtration system is a microfiltration system configured for separating clonal phages from particles larger the phages, such as bacterial cells and debris. |
| P73. | The phage system according to at least one of P69 to P72, wherein the compartment configured for phage harvesting comprises a size-exclusion chromatography system configured for purifying and separating clonal phages from smaller or larger contaminants based on size. |
| P74. | The phage system according to at least one of P69 to P73 further comprising at least one clonal phage collecting container. |
| P75. | The phage system according to at least one of P70 to P73 wherein the compartment configured for phage harvesting further comprises an outlet configured for feeding back the retentate to the compartment of phage production. |
| P76. | The phage system according to any one of preceding embodiments, wherein the phage system comprises a control unit configured to monitor and/or control pumps and valves, monitor pH, temperature etc. |
| P77. | The phage system according to P76 wherein the pumps are configured for pumping liquids through the system or parts thereof. |
| P78. | The phage system according to P76 and/or P77 wherein the pumps comprise syringe pumps and peristaltic pumps of a size depending on the volume of the liquids to be pumped. |
| P79. | The phage system according to P78 wherein the syringe pumps are configured to pump dosage amounts in the range of 10 to 2500 µl for dosing bacteria growth media, bacterial cell source and phage samples. |
| P80. | The phage system according to P78 wherein the peristaltic pumps are configured to pump greater than 2500 µl volumes of liquid. |
| P81. | The phage system according to P80, wherein the greater than 2500 µl volumes pumped comprise water, cleaning solution (NaOH), waste and phage product. |
| P82. | The phage system according to at least one of P76 to P81 wherein the valves are configured for controlling the flow of the liquid media and/or the volume thereof within one module and through the different modules, units and compartments. |
| P83. | The phage system according to P82 wherein the liquid media comprises bacteria growth media, bacterial cell source and phage samples from the phage enrichment compartment, the isolation module and the phage production compartment. |
| P84. | The phage system according to at least one of P82 to P83 wherein the valves are configured to be opened or closed upon a corresponding signal of the control unit. |
| M1. | The method of continuously and automatically isolating at least one clonal population of phages from a phage source containing a mixture of phages or a phage source containing one single phage type. |
| M2. | The method according to M1 wherein the phage source containing a mixture of phages used is liquid. |
| M3. | The method according to M2 wherein the liquid phage rich sample used is a mixture of natural phages, artificial phages or combinations thereof. |
| M4. | The method according to the M2 and/or M3 wherein the liquid phage rich sample used is a mixture of known phages or unknown phages or combinations thereof. |
| M5. | The method according to at least any one M1 to M4 wherein an environmental, laboratory stock or clinical sample is used as a phage rich source. |
| M6. | The method according to M5 wherein a wastewater sample is used as an environmental sample. |
| M7. | The method according to M6 wherein the wastewater sample is filtered before being used as a phage source. |
| M8. | The method according to M7 wherein one or more dead-end filters are used. |
| M9. | The phage system according to M8, wherein the filter/s have pore size of 0.10 µm to 1.0 µm. |
| M10. | The phage system according to M9, wherein the filter/s have a pore size of 0.22 µm to 0.45 µm. |
| M11. | The phage system according to M10, wherein the filter/s have a pore size of 0.22 µm. |
| M12. | The phage system according to M8 to M11 wherein the filters are connected in series. |
| M13. | The method according to at least one of M1 to M12 wherein the filtered phage source containing a mixture of phages is subjected to a phage enrichment process. |
| M14. | The method according to M13 wherein the phage source is added to at least one phage enrichment container containing at least one bacterial culture in a bacteria culture medium. |
| M15. | The method according to M14 wherein 2 to 10 phage enrichment containers containing each different bacterial culture are used. |
| M16. | The method according to at least any one of M13 to M15 wherein the phages are grown on the bait bacteria host cells in the at least one phage enrichment container while the concentration of the bacteria is monitored. |
| M17. | The method according to M16, wherein the phage enrichment is carried out in more than one container. |
| M18. | The method according to M17 wherein the at least one bacterial culture is grown in the at least one bacteria growth container. |
| M19. | The method according to M18 wherein the bacteria culture is grown to exponential or stationary phase. |
| M20. | The method according to at least one of M14 to M19 wherein bacteria selected from bacteria isolates from diagnostic laboratories of veterinarian and human origin, or from environmental, agricultural, industrial, food industry, animal care, plant-associated sources, GMO strains, strain collections or combinations thereof are used as bacterial cells. |
| M21. | The method according to M20 wherein the growth of the bacteria is kept in steady state culture density by adding fresh growth media while the outgrown bacterial culture is transferred to other containers and/or isolation modules. |
| M22. | The method according to M21 wherein the bacteria are transferred continuously or as a batch at a desired concentration. |
| M23. | The method according to at least one of M14 to M22 wherein the concentration of the bacteria is measured by a bacteria quantifying means. |
| M24. | The method according to M12 wherein the concentration of the bacteria is measured by physical or chemical detection methods selected from OD measurement, calorimetric measurement, LED measurement, light interaction fluorescence, flow cytometry, Nanoparticle Tracking Analysis, photo diode measurement and/or visual microscope image analysis. |
| M25. | The method according to at least one of M23 and M24, wherein OD monitoring measures bacterial cell density in real time by detecting light scattering, and a steep decline following phage addition indicates high infection efficiency, a short latency period, and a large burst size, leading to effective amplification of the phage population. |
| M26. | The method according to M25 wherein the bacteria are quantified by OD measurement, and the phages selected for use and inclusion in phage biobanks demonstrate rapid and stable bacterial lysis, observable as a sharp and sustained decrease in OD shortly after infection. |
| M27. | The method according to M25, wherein sustained low OD values following phage-induced lysis are used as indicators of efficient adsorption, replication, and host cell destruction, and are interpreted as evidence of robust lytic pressure and limited bacterial regrowth or resistance development. |
| M28. | The method according to at least one of the preceding embodiments, wherein phages that result in delayed, partial, or inconsistent OD reductions, exhibit instability over time, or allow early regrowth of bacterial populations, are excluded from therapeutic collections, and wherein OD monitoring is used as a real-time, quantitative tool for screening, characterizing, and prioritizing phage candidates or phage mixtures based on biological performance and clinical relevance. |
| M29. | The method according to M24 wherein the bacteria are quantified by OD measurement. |
| M30. | The method according to M29 wherein phage enrichment is completed when a reduced growth rate of bacteria due to phage lysis in the medium is observed. |
| M31. | The method according to at least one of M13 to M30 wherein the filtered enriched phage mixture is subjected to a technique for clonal isolation of a phage population from a phage mixture. |
| M32. | The method according to M31 wherein the phage enriched mixture from the phage enrichment process is filtered before clonal isolation of a phage population using one or more dead end filters as defined in at least one of M8 to M12. |
| M33. | The method according to at least one of M31 to M32 wherein the filtered enriched phage mixture is subjected to a limited dilution series in an isolation module. |
| M34. | The method according to M33 wherein a plate having 24 to 1536 wells is used for isolation of the phage population. |
| M35. | The method according to M34 wherein a plate having 96 or 384 wells is used. |
| M36. | The method according to at least one of M33 to M35 wherein the enriched phage mixture is distributed onto the wells by a pipette. |
| M37. | The method according to M36 wherein bacteria culture cells are added to the wells from a bacteria growth container as defined in at least one of M22 to M26 allowing the interaction between phages and bacteria to take place. |
| M38. | The method according to M37 wherein the phages and the bacteria are incubated in the wells and the concentration of the bacteria in the limited dilution series is continuously measured in the wells. |
| M39. | The method according to M38 wherein the concentration of bacteria is measured in the wells using a bacteria quantifying means as defined in at least one of M23 to M29. |
| M40. | The method according to M39 wherein the phage sample with the most diluted sample which still results in lysis selected. |
| M41. | The method according to M40 wherein the phage sample with the most diluted sample is subjected to another round of limited dilution series and monitored using a bacteria quantifying means as defined in at least one of M23 to M29. |
| M42. | The method according to M41 wherein the limited dilution series is repeated until the most diluted sample that still presents bacterial lysis can be identified. |
| M43. | The method according to M42 wherein the most diluted sample of the last repetition is isolated. |
| M44. | The method according to M31 and/or M32, wherein a digital microfluidic device configured to create, merge, and split droplets using electrowetting on dielectric is used for isolating the phage population, wherein droplets containing phages are serially diluted by splitting and subsequently merged with droplets containing bacterial cells, buffer, or media, and wherein the resulting droplets can be further processed directly on-chip or transferred to a pipetting robot, microplate, or plate reader unit for downstream processing. |
| M45. | The method according to M31 and/or M32 wherein a continuous flow microfluidics chip is used for isolating the phage population, wherein the chip comprises separate inputs for introducing phage mixtures and media, bacterial host cells, or buffers, and wherein channels within the chip merge into a tree structure enabling gradual dilution and mixing by diffusion or turbulence, each branch of the tree structure providing different dilution levels determined by flow rates, wherein bacterial host cells can be supplied to each branch and bacterial growth monitored through optical density (OD) measurements performed on-chip as defined in any one of M23 and/or M24, and wherein liquids from each branch are optionally transferred to isolation module/s according to P37 to P60. |
| M46. | The method according to M45 wherein the diluted phage in liquid is mixed with bacterial cells and measured on the chip for bacterial growth in a parallel or serial way whereby the lowest dilution which results in lysis is filtered and returned to the production unit. |
| M47. | The method according to M46 wherein the measurement of bacteria concentration on the chip is performed as defined in one of at least M23 to M25. |
| M48. | The method according to at least one of M32 to M47 wherein the sample with the isolated phage population is transferred to at least one phage production container. |
| M49. | The method according to M48 wherein the isolated phage sample is filtered before entering the at least one phage production container as defined in at least one of M8 to M12. |
| M50. | The method according to at least one of M48 to M49 wherein phages from the isolated phage population are grown in the presence of host bacterial cells in the production container. |
| M51. | The method according to M50 wherein the isolated phages are grown on the host bacterial cells in an exponential or stationary culture. |
| M52. | The method according to M51 wherein bacteria from at least one bacteria growth container are added to the phage production container as defined in at least one of M18 to M22. |
| M53. | The method according to M52 wherein the addition of bacteria is monitored by continuously measuring the concentration of the bacteria as defined in at least one of M23 to M29. |
| M54. | The method according to M53 wherein the phages are harvested when the OD has reached a predetermined range. |
| M55. | The method according to M54 wherein the phages are harvested via tangential flow filtration. |
| M56. | The method according to M54 wherein the phages are harvested by using one or more dead end filters as defined in at least one of M8 to M12. |
| M57. | The method according to at least one of M54 to M56 wherein the filtered phage permeate is collected in a vessel. |
| M58. | The method according to M57 wherein the retentate is fed back to the phage production container. |
| M59. | The method according to M58 wherein the phages are further clonally purified by recycling same to the phage enrichment container. |
| M60. | The method according to M59 wherein the phages are recycled by passing through one or more dead end filters as defined in M8 to M12. |
| M61. | The method according to at least one of M55 to M60 wherein the harvested phages are up concentrated by lowering the volume for the phages. |
| M62. | The method according to at least one of M1 to M61 wherein the harvest volume is 200-5000 ml/day. |
| M63. | The method according to at least one of M1 to M62 which is run in a phage production system. |
| M64. | The method according to M63, wherein continuous fermentation is performed with machine- controlled dynamic dilution rates of phages and bacterial cells, enabling optimized and stabilized measurement of phage-host interaction parameters including latency period, burst size, multiplicity of infection (MOI) effects, phage stability, synergy, and resistance dynamics. |
| M65. | The method according to at least one of the preceding embodiments, wherein controlled continuous fermentation simulates application-relevant environments to improve phage selection, enabling development of clinically effective phage biobanks and supporting rapid infection control. |
| M66. | The method according to M65 wherein the system is cleaned with a cleaning process that begins with a 1 to 5% NaOH flush, and then followed by water to remove residues and eliminate any remaining cleaning solution, with all fluids directed to waste to prevent backflow and contamination. |
| M67. | The method according to M66 wherein only parts of the system are cleaned and the remaining part continues to operate. |
| U1. | Use of at least one clonal phage population isolate, including its virions, produced according to at least one of M1 to M67 and the derivatives thereof, in the preparation of a phage biobank, wherein the phage biobank comprises a collection of characterized phage isolates and/or virions stored for future applications. |
| U2. | Use according to U1, wherein the phage biobank further comprises phage-derived substances and information. |
| U3. | Use according to U2, wherein the phage-derived substances comprise proteins, including enzymes such as endolysins and holins. |
| U4. | Use according to at least one of U1 to U3, wherein the phage-derived information comprises genomic information, including nucleic acid sequences. |
| U5. | Use according to at least one of U1 to U3, wherein the phage-derived information comprises phage phenotypical characteristics. |
| U6. | Use according to at least one of U1 to U5, in the preparation of pharmaceutical active ingredients in the field of human and veterinary medicine. |
| U7. | Use according to U6, in the preparation of a medical device. |
| U8. | Use according to at least one of U1 to U5, in research and development. |
| U9. | Use according to at least one of U1 to U5, in the manufacture of phages for various industrial applications. |
| U10. | Use according to U9, in the food industry, agriculture, farming, chemical industry, animal care, cosmetics, water management, space travel, air purification, decontamination of areas, and in sanitation and hygiene. |
| U11. | Use according to U10, in the preparation of a biocide, a food supplement, a feed supplement, a feed additive, an animal care product, a human cosmetic, a processing aid, a food additive, and/or a preservative for pathogen control. |
| U12. | Use according to U1 or U2, in diagnostics outside the human and animal body. |
| U13. | Use according to U1 or U2, in biotechnology. |
| U14. | An isolate comprising at least one clonal phage population isolate produced according to at least one of M1 to M67, the derivatives thereof, and phage-derived substances and information, for use in prophylactic therapy, metaphylactic therapy, personalized therapy, non-personalized therapy, and/or fit-for-purpose therapy of individuals suffering from infectious diseases caused by bacteria. |
| U15. | The phage isolate, its derivatives, and phage-derived substances and information according to U14, wherein the individuals are humans or animals. |
| U16. | The phage isolate, its derivatives, and phage-derived substances and information according to U14 or U15, wherein the bacteria targeted are antibiotic-resistant bacteria and/or bacteria causing infections. |
| U17. | The phage isolate, its derivatives, and phage-derived substances and information according to U16, wherein the bacteria resistant to antibiotics are selected from carbapenem-resistant *Klebsiella pneumoniae, Escherichia coli,* and *Acinetobacter baumannii*; penicillin-resistant Group B Streptococci; macrolide-resistant Group A Streptococci; methicillin-resistant *Staphylococcus aureus*; and third-generation cephalosporin-resistant *Enterobacter* species. |
| U18. | The phage isolate, its derivatives, and phage-derived substances and information according to U16, wherein the bacteria causing infections are selected from *Staphylococcus spp., Proteus spp., Escherichia coli, Klebsiella spp.,* and *Pseudomonas aeruginosa* in companion animals, as well as *Staphylococcus aureus, Enterococcus spp., Salmonella spp.,* and *Clostridium spp.* in food-producing animals. |

### Brief description of drawings

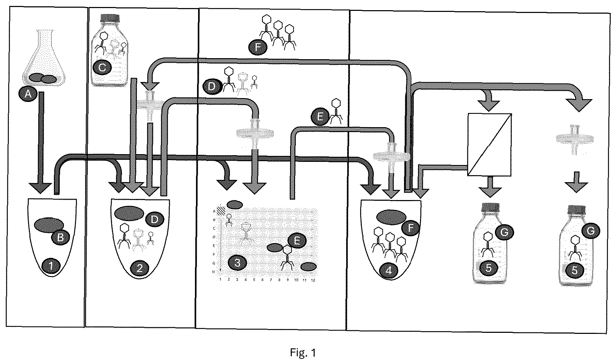

Fig. 1 illustrates a schematic diagram of a phage production system and method for the continuous and automated production and isolation of phages.

## Claims

1. A phage system, for continuous production of clonal phages, the phage system comprising a bioreactor, wherein the bioreactor comprises:
• at least one compartment configured for culturing bacterial cells (1), wherein the compartment for culturing bacterial cells comprises at least one inlet for introducing growth media and/or bacterial cell source (A) and at least one outlet for transferring the cultured bacterial cells (B) from the compartment configured for culturing bacterial cells (1), and
• at least one compartment configured for phage enrichment (2), wherein the compartment configured for phage enrichment (2) comprises at least one inlet configured for introducing an initial population of phages (C), growth media and/or replenishing bacterial cells (B) and at least one outlet for transferring an enriched mixture of phages (D) and bacteria (B) from the compartment configured for phage enrichment (2),
**characterised in that** the phage system further comprises:
• at least one isolation module (3), wherein the isolation module (3) comprises at least one inlet configured for introducing the enriched mixture of phages (D) and at least one outlet for transferring isolated phages (E) from the isolation module (3) and wherein the isolation module (3) is configured to perform serial dilution of the enriched mixture to single out and isolate a clonal phage, wherein the isolated phage is identified as the most diluted sample from the final repetition that still results in lysis,
• at least one monitoring system that enables measurement of bacterial growth or lysis, and comprises pumps configured for pumping liquid media through the system or parts thereof, and valves configured to be opened or closed, wherein the pumps and valves are controlled by signals from a control unit, and wherein the control unit is further configured to monitor the operational status of the pumps and valves, as well as at least one of temperature, pH, and nutrient supply, and
• one or more sensor units configured to monitor and/or record phage characteristics, including lysis events, during the phage harvesting process, wherein the recorded data is used for characterization of the phages and/or preparation of a phage biobank, wherein the characterization comprises machine-controlled dynamic dilution rates of phages and bacterial cells and stabilized measurement of phage-host interaction parameters including latency period, burst size, multiplicity of infection (MOI) effects, phage stability, synergy and resistance dynamics.

2. The phage system according to claim 1, wherein the phage system further comprises at least one compartment configured for phage production (4) configured for phage clonal expansion and wherein the compartment for phage production (4) comprises at least one inlet for introducing growth media, bacterial cell source (B) or clonal population of phages (E) from the isolation module (3) and at least one outlet for transferring phages from the compartment configured for phage production (4).

3. The phage system according to claim 2, wherein the phage system further comprises at least one compartment configured for phage harvesting (5), wherein the compartment configured for phage harvesting (5) comprises at least one inlet configured for introducing expanded clonal population of phages (F) from the compartment configured for phage production (4) and at least one outlet for collecting the phages from the compartment configured for phage harvesting (5).

4. The phage system according to any one of preceding claims, wherein the at least one isolation module is configured to perform a limited dilution series of bacteriophages until a dilution of 10^-14.

5. The phage system according to claim 3, wherein the compartment configured for phage harvesting comprises a tangential flow filtration unit and/or size-exclusion chromatography unit configured for purifying and separating clonal phages.

6. The phage system according to any one of claims 1 to 5, wherein the isolation module (3) comprises a digital microfluidic device using electrowetting on dielectric configured to create, merge, and manipulate droplets, and wherein one or several droplets generated on the digital microfluidic device allow for a serial dilution by fission of phage-containing droplets and merging them with cell, buffer, or media-containing droplets.

7. The phage system according to any one of claims 1 to 6, wherein the monitoring system is configured to measure bacterial growth or lysis in real time based on optical density (OD), wherein phage growth is inferred from a decrease in live bacterial concentration, and a steep OD decline is indicative of rapid and stable phage-induced bacterial lysis.

8. The phage system according to any one of claims 1 to 7, wherein the phage system comprises a compartment configured for phage harvesting (5) comprising a tangential flow filtration unit configured to separate clonal phages from bacterial cells and culture medium, and wherein the compartment comprises an outlet configured for feeding back the retentate to the compartment configured for phage production (4).

9. A method of continuously producing clonal phages, the method comprising the steps of:
• culturing bacterial cells (B) in at least one compartment configured for culturing bacterial cells (1) by introducing growth media and a bacterial cell source (A) into the compartment configured for culturing bacterial cells (1) and transferring the cultured bacterial cells from the compartment configured for culturing bacterial cells (1),
• enriching phages in at least one compartment configured for phage enrichment (2) by introducing an initial population of phages (C), growth media and/or bacterial cells (A) into the compartment configured for phage enrichment (2) and transferring the resulting enriched mixture of phages and bacterial cells from the compartment configured for phage enrichment (2),
• isolating clonal phages from the enriched mixture of phages in at least one isolation module (3) by introducing the enriched mixture of phages into the isolation module (3),
**characterised in that** the method further comprises:
performing a limited dilution series of the enriched mixture of phages (D) in the isolation module (3) using at least one of a pipetting robot, a digital microfluidic system or a gradient microfluidic chip and selecting the most diluted sample with observable bacterial lysis for clonal expansion.

10. The method of continuously producing clonal phages according to claim 9, wherein the filtered enriched phage mixture is subjected to a limited dilution series in the isolation module (3).

11. The method of continuously producing clonal phages (F) according to any one of claims 9 to 10, wherein the enriched mixture of phages (D) is distributed into at least one plate having 24 to 1536 wells for isolation of the phage population in the isolation module (3).

12. The method of continuously producing clonal phages according to any one of claims 9 to 11, wherein the phages (D) and the bacteria (A) are incubated in the wells and the concentration of the bacteria (A) in the limited dilution series is continuously measured in the wells.

13. The method according to any one of claims 9 to 12, wherein retentate from the phage harvesting process is fed back to the compartment configured for phage production and wherein the phages are further clonally purified by recycling the harvested phage sample to the phage enrichment compartment through one or more dead-end filters.

14. Use of at least one clonal phage population isolate (F), produced according to any one of claims 9 to 13, or a derivative thereof, for preparing a phage biobank comprising a collection of characterized phage isolates (F) for future applications.

15. An isolate comprising at least one clonal phage population isolate produced according to at least one of claims 9 to 13, the derivatives thereof, and phage-derived substances and information, for use in prophylactic therapy, metaphylactic therapy, personalized therapy, non-personalized therapy, and/or fit-for-purpose therapy of individuals or animals suffering from infectious diseases caused by bacteria, and/or for use in veterinary medicine, agriculture, environmental sanitation, diagnostics, or industrial applications.
